# EUROPEAN PATENT APPLICATION

(11) **EP 4 001 404 A1**
(43) Date of publication of application: **25.05.2022**
(21) Application number: 20839589.7
(22) Date of filing: 04.06.2020
(51) Int. Cl.: C12N 7/01, C12N 15/57, A61K 35/763, A61K 35/768, A61P 35/00

(54) **VIRUS AND TUMOR THERAPEUTIC DRUG FOR SPECIFICALLY KILLING TUMOR CELLS**

(30) Priority: 16.07.2019 CN 201910644539
(71) Applicant: Wu, Zetang, Hefei, Anhui 230088 (CN)
(72) Inventor: Wu, Zetang, Hefei, Anhui 230088 (CN)
(74) Representative: Kailuweit & Uhlemann Patentanwälte Partnerschaft mbB
(86) International application number: PCT/CN2020/094435
(87) International publication number: WO 2021/008267

(57) **Abstract**

Provided are a virus and a tumor therapeutic drug for specifically killing tumor cells. The virus is a recombinant oncolytic virus, and the genome thereof has an exogenous promoter inserted which is located upstream of an essential gene of the virus to replace the exogenous promoter of the essential gene, and to drive the expression of the essential gene in tumor cells but not in normal cells. The virus can kill a variety of tumor cells with an efficacy similar to that of the wild-type virus while it is safe to non-tumor cells. *In vivo* studies indicate that the oncolytic viruses provided in this disclosure can significantly inhibit tumor growth in various tumor animal models.

SEQUENCE LISTING

<110>  ZETANG WU

<120>  Virus and Tumor Therapeutic Drug for Specifically Killing Tumor Cells

<130>  OPI21301524EPO

<150>  CN2019106445393
<151>  2019-07-16

<150>  PCT/CN2020/094435
<151>  2020-06-04

<160>  5

<170>  PatentIn version 3.5

<210>  1
<211>  2679
<212>  DNA
<213>  Artificial sequence

<220>
<223>  Nucleotide sequence of the first nucleic acid fragment

<400>  1
   aaataactcg agctgcgggc gctgttgttc catcatcctg tcgggcatcg caatgcgatt      60
   gtgttatatc gccgtggtgg ccggggtggt gctcgtggcg cttcactacg agcaggagat     120
   ccagaggcgc ctgtttgatg tatgacgtca catccaggcc ggcggaaacc ggaacggcat     180
   atgcaaactg gaaactgtcc tgtcttgggg cccacccacc cgacgcgtca tatgtaaatg     240
   aaaatcgttc ccccgaggcc atgtgtagcc tggatcccaa cgaccccgcc catgggtccc     300
   aattggccgt cccgttacca agaccaaccc agccagcgta tccacccccg cccgggtccc     360
   cgcggaagcg gaacggtgta tgtgatatgc taattaaata catgccacgt acttatggtg     420
   tctgattggt ccttgtctgt gccggaggtg gggcgggggc cccgcccggg gggcggaact     480
   aggaggggtt tgggagagcc ggccccggca ccacgggtat aaggacatcc accacccggc     540
   cggtggtggt gtgcagccgt gttccaacca cggtcgttga cattgattat tgactagtta     600
   ttaatagtaa tcaattacgg ggtcattagt tcatagccca tatatggagt tccgcgttac     660
   ataacttacg gtaaatggcc cgcctggctg accgcccaac gacccccgcc cattgacgtc     720
   aataatgacg tatgttccca tagtaacgcc aatagggact ttccattgac gtcaatgggt     780
   ggactattta cggtaaactg cccacttggc agtacatcaa gtgtatcata tgccaagtac     840
   gccccctatt gacgtcaatg acggtaaatg gcccgcctgg cattatgccc agtacatgac     900
   cttatgggac tttcctactt ggcagtacat ctacgtatta gtcatcgcta ttaccatggt     960
   gatgcggttt tggcagtaca tcaatgggcg tggatagcgg tttgactcac ggggatttcc    1020
   aagtctccac cccattgacg tcaatgggag tttgttttgg caccaaaatc aacgggactt    1080
   tccaaaatgt cgtaacaact ccgccccatt gacgcaaatg ggcggtaggc gtgtacggtg    1140
   ggaggtctat ataagcagag ctctctggct aactgccacc atggtgagca agggcgagga    1200
   gctgttcacc ggggtggtgc ccatcctggt cgagctggac ggcgacgtaa acggccacaa    1260
   gttcagcgtg tccggcgagg gcgagggcga tgccacctac ggcaagctga ccctgaagtt    1320
   catctgcacc accggcaagc tgcccgtgcc ctggcccacc ctcgtgacca ccctgaccta    1380
   cggcgtgcag tgcttcagcc gctaccccga ccacatgaag cagcacgact tcttcaagtc    1440
   cgccatgccc gaaggctacg tccaggagcg caccatcttc ttcaaggacg acggcaacta    1500
   caagacccgc gccgaggtga agttcgaggg cgacaccctg gtgaaccgca tcgagctgaa    1560
   gggcatcgac ttcaaggagg acggcaacat cctggggcac aagctggagt acaactacaa    1620
   cagccacaac gtctatatca tggccgacaa gcagaagaac ggcatcaagg tgaacttcaa    1680
   gatccgccac aacatcgagg acggcagcgt gcagctcgcc gaccactacc agcagaacac    1740
   ccccatcggc gacggccccg tgctgctgcc cgacaaccac tacctgagca cccagtccgc    1800
   cctgagcaaa gaccccaacg agaagcgcga tcacatggtc ctgctggagt tcgtgaccgc    1860
   cgccgggatc actctcggca tggacgagct gtacaagtaa cgggcctcga ctgtgccttc    1920
   tagttgccag ccatctgttg tttgcccctc ccccgtgcct tccttgaccc tggaaggtgc    1980
   cactcccact gtcctttcct aataaaatga ggaaattgca tcgcattgtc tgagtaggtg    2040
   tcattctatt ctggggggtg gggtggggca ggacagcaag ggggaggatt gggaagacaa    2100
   tagcaggcat gctggggatg cggtgggctc tatggcttct gaggtacaat aaaaacaaaa    2160
   catttcaaac aaatcgcccc acgtgttgtc cttctttgct catggccggc ggggcgtggg 2220
   tcacggcaga tggcgggggt gggcccggcg tacggcctgg gtgggcggag ggaactaacc 2280
   caacgtataa atccgtcccc gctccaaggc cggtgtcata gtgcccttag gagcttcccg 2340
   cccgggcgca tccccccttt tgcactatga cagcgacccc cctcaccaac ctgttcttac 2400
   gggccccgga cataacccac gtggcccccc cttactgcct caacgccacc tggcaggccg 2460
   aaacggccat gcacaccagc aaaacggact ccgcttgcgt ggccgtgcgg agttacctgg 2520
   tccgcgcctc ctgtgagacc agcggcacaa tccactgctt tttctttgcg gtatacaagg 2580
   acacccacca tacccctccg ctgattaccg agctccgcaa ctttgcggac ctggttaacc 2640
   acccgccggt cctacgcgaa ctggtcgaag ctttttata 2679

<210> 2
<211> 3582
<212> DNA
<213> Artificial sequence

<220>
<223> Nucleotide sequence of the second nucleic acid fragment

<400> 2
ctcgaggtca tcgtaggctg cgagttgatg ctacgctttg tggccgtggg tctcatcgtc 60
ggcaccgctt tcatatcccg gggggcatgt gcgatcacat accccctgtt tctgaccatc 120
accacctggt gttttgtctc caccatcggc ctgacagagc tgtattgtat tctgcggcgg 180
ggcccggccc ccaagaacgc agacaaggcc gccgccccgg ggcgatccaa ggggctgtcg 240
ggcgtctgcg ggcgctgttg ttccatcatc ctgtcgggca tcgcaatgcg attgtgttat 300
atcgccgtgg tggccggggt ggtgctcgtg gcgcttcact acgagcagga gatccagagg 360
cgcctgtttg atgtatgacg tcacatccag gccggcggaa accggaacgg catatgcaaa 420
ctggaaactg tcctgtcttg gggcccaccc acccgacgcg tcatatgtaa atgaaaatcg 480
ttcccccgag gccatgtgta gcctggatcc caacgacatg gcccctccct cgggttaccc 540
cacagcctag gccgattcga cctctctccg ctggggccct cgctggcgtc cctgcaccct 600
gggagcgcga gcggcgcgcg ggcggggaag cgcggcccag acccccgggt ccgcccggag 660
cagctgcgct gtcggggcca ggccgggctc ccagtggatt cgcgggcaca gacgcccagg 720
accgcgctcc ccacgtggcg gagggactgg ggacccgggc acccgtcctg ccccttcacc 780
ttccagctcc gcctcctccg cgcggacccc gccccgtccc gacccctccc gggtccccgg 840
cccagccccc tccgggccct cccagcccct ccccttcctt tccgcggccc cgccctctcc 900
tcgcggcgcg agtttcaggc agcgctgcgt cctgctgcgc acgtgggaag ccctggcccc 960
ggccaccccc gcgtaggcgt gtacggtggg aggcctatat aagcagagct cgtttagtga 1020
accgtcagat cgcctggaga tggcgactga cattgatatg ctaattgacc tcggcctgga 1080
cctctccgac agcgatctgg acgaggaccc ccccgagccg gcggagagcc gccgcgacga 1140
cctggaatcg gacagcaacg gggagtgttc ctcgtcggac gaggacatgg aagaccccca 1200
cggagaggac ggaccggagc cgatactcga cgccgctcgc ccggcggtcc gcccgtctcg 1260
tccagaagac cccggcgtac ccagcaccca gacgcctcgt ccgacggagc ggcagggccc 1320
caacgatcct caaccagcgc cccacagtgt gtggtcgcgc ctcggggccc ggcgaccgtc 1380
ttgctccccc gagcggcacg ggggcaaggt ggcccgcctc caacccccac cgaccaaagc 1440
ccagcctgcc cgcggcggac gccgtgggcg tcgcaggggt cggggtcgcg gtggtcccgg 1500
ggccgccgat ggtttgtcgg acccccgccg gcgtgccccc agaaccaatc gcaacccggg 1560
gggaccccgc cccggggcgg ggtggacgga cggccccggc gccccccatg gcgaggcgtg 1620
gcgcggaagt gagcagcccg acccacccgg aggcccgcgg acacggagcg tgcgccaagc 1680
accccccccg ctaatgacgc tggcgattgc ccccccgccc gcggaccccc gcgccccggc 1740
cccggagcga aaggcgcccg ccgccgacac catcgacgcc accacgcggt tggtcctgcg 1800
ctccatctcc gagcgcgcgg cggtcgaccg catcagcgag agcttcggcc gcagcgcaca 1860
ggtcatgcac gacccctttg gggggcagcc gtttcccgcc gcgaatagcc cctgggcccc 1920
ggtgctggcg ggccaaggag ggccctttga cgccgagacc agacgggtct cctgggaaac 1980
cttggtcgcc cacggcccga gcctctatcg cacttttgcc ggcaatcctc gggccgcatc 2040
gaccgccaag gccatgcgcg actgcgtgct gcgccaagaa aatttcatcg aggcgctggc 2100
ctccgccgac gagacgctgg cgtggtgcaa gatgtgcatc caccacaacc tgccgctgcg 2160
cccccaggac cccattatcg ggacggccgc ggcggtgctg gataacctcg ccacgcgcct 2220
gcggcccttt ctccagtgct acctgaaggc gcgaggcctg tgcggcctgg acgaactgtg 2280
ttcgcggcgg cgtctggcgg acattaagga cattgcatcc ttcgtgtttg tcattctggc 2340
caggctcgcc aaccgcgtcg agcgtggcgt cgcggagatc gactacgcga cccttggtgt 2400
cggggtcgga gagaagatgc atttctacct ccccggggcc tgcatggcgg gcctgatcga 2460
aatcctagac acgcaccgcc aggagtgttc gagtcgtgtc tgcgagttga cggccagtca 2520
catcgtcgcc cccccgtacg tgcacggcaa atatttttat tgcaactccc tgttttagag 2580
acatgataag atacattgat gagtttggac aaaccacaac tagaatgcag tgaaaaaaat 2640

gctttatttg tgaaatttgt gatgctattg ctttatttgt aaccattata agctgcaata 2700
aacaagttaa caacaacaat tgcattcatt ttatgtttca ggttcagggg gaggtgtggg 2760
aggtttttta aagcaagtaa aacctctaca aatgtggtat ggctgattat gatcctgcaa 2820
gcctcgtcgt cctggccgga ccacgctatc tgtgcaaggt ccccggcccc ggacgcgcgc 2880
tccatgagca gagcgcccgc cgccgaggcg aagactcggg cggcgccctg cccgtcccac 2940
caggtcaaca ggcggtaacc ggcctcttca tcgggaatgc gcgcgacctt cagcatcgcc 3000
ggcatgtccc cctggcggac gggaagtatc cagctcgacc aagctgtttt aagcttgtac 3060
aataaaaaca aaacatttca aacaaatcgc cccacgtgtt gtccttcttt gctcatggcc 3120
ggcggggcgt gggtcacggc agatggcggg ggtgggcccg gcgtacggcc tgggtgggcg 3180
gagggaacta acccaacgta taaatccgtc cccgctccaa ggccggtgtc atagtgccct 3240
taggagcttc ccgcccgggc gcatcccccc ttttgcacta tgacagcgac ccccctcacc 3300
aacctgttct tacgggcccc ggacataacc cacgtggccc ccccttactg cctcaacgcc 3360
acctggcagg ccgaaacggc catgcacacc agcaaaacgg actccgcttg cgtggccgtg 3420
cggagttacc tggtccgcgc ctcctgtgag accagcggca caatccactg ctttttcttt 3480
gcggtataca aggacaccca ccatacccct ccgctgatta ccgagctccg caactttgcg 3540
gacctggtta accacccgcc ggtcctacgc gaactgctcg ag 3582

<210> 3
<211> 2310
<212> DNA
<213> Artificial sequence

<220>
<223> Nucleotide sequence of the third nucleic acid fragment

<400> 3
aagcttatgg cccctccctc gggttacccc acagcctagg ccgattcgac ctctctccgc 60
tggggccctc gctggcgtcc ctgcaccctg ggagcgcgag cggcgcgcgg gcggggaagc 120
gcggcccaga cccccgggtc cgcccggagc agctgcgctg tcggggccag gccgggctcc 180
cagtggattc gcgggcacag acgcccagga ccgcgctccc cacgtggcgg agggactggg 240
gacccgggca cccgtcctgc cccttcacct tccagctccg cctcctccgc gcggaccccg 300
ccccgtcccg acccctcccg ggtccccggc ccagccccct ccgggccctc ccagcccctc 360
cccttccttt ccgcggcccc gccctctcct cgcggcgcga gtttcaggca gcgctgcgtc 420
ctgctgcgca cgtgggaagc cctggccccg gccacccccg cggtaggcgt gtacggtggg 480
aggcctatat aagcagagct cgtttagtga accgtcagat cgcctggaat ggcgactgac 540
attgatatgc taattgacct cggcctggac ctctccgaca gcgatctgga cgaggacccc 600
cccgagccgg cggagagccg ccgcgacgac ctggaatcgg acagcaacgg ggagtgttcc 660
tcgtcggacg aggacatgga agacccccac ggagaggacg gaccggagcc gatactcgac 720
gccgctcgcc cggcggtccg cccgtctcgt ccagaagacc ccggcgtacc cagcacccag 780
acgcctcgtc cgacggagcg gcagggcccc aacgatcctc aaccagcgcc ccacagtgtg 840
tggtcgcgcc tcggggcccg gcgaccgtct tgctcccccg agcggcacgg gggcaaggtg 900
gcccgcctcc aacccccacc gaccaaagcc cagcctgccc gcggcggacg ccgtgggcgt 960
cgcaggggtc ggggtcgcgg tggtcccggg gccgccgatg gtttgtcgga cccccgccgg 1020
cgtgccccca gaaccaatcg caacccgggg ggaccccgcc ccgggcgggg gtggacggac 1080
ggccccggcg ccccccatgg cgaggcgtgg cgcggaagtg agcagcccga cccacccgga 1140
ggcccgcgga cacggagcgt gcgccaagca ccccccccgc taatgacgct ggcgattgcc 1200
cccccgcccg cggacccccg cgccccggcc ccggagcgaa aggcgcccgc cgccgacacc 1260
atcgacgcca ccacgcggtt ggtcctgcgc tccatctccg agcgcgcggc ggtcgaccgc 1320
atcagcgaga gcttcggccg cagcgcacag gtcatgcacg acccctttgg ggggcagccg 1380
tttcccgccg cgaatagccc ctgggccccg gtgctggcgg gccaaggagg gccctttgac 1440
gccgagacca gacgggtctc ctgggaaacc ttggtcgccc acggcccgag cctctatcgc 1500
acttttgccg gcaatcctcg ggccgcatcg accgccaagg ccatgcgcga ctgcgtgctg 1560
cgccaagaaa atttcatcga ggcgctggcc tccgccgacg agacgctggc gtggtgcaag 1620
atgtgcatcc accacaacct gccgctgcgc ccccaggacc ccattatcgg gacggccgcg 1680
gcggtgctgg ataacctcgc cacgcgcctg cggccctttc tccagtgcta cctgaaggcg 1740
cgaggcctgt gcggcctgga cgaactgtgt tcgcggcggc gtctggcgga cattaaggac 1800
attgcatcct tcgtgtttgt cattctggcc aggctcgcca accgcgtcga gcgtggcgtc 1860
gcggagatcg actacgcgac ccttggtgtc ggggtcggag agaagatgca tttctacctc 1920
cccggggcct gcatggcggg cctgatcgaa atcctagaca cgcaccgcca ggagtgttcg 1980
agtcgtgtct gcgagttgac ggccagtcac atcgtcgccc ccccgtacgt gcacggcaaa 2040
tatttttatt gcaactccct gttttaggac tgtgccttct agttgccagc catctgttgt 2100
ttgcccctcc cccgtgcctt ccttgaccct ggaaggtgcc actcccactg tcctttccta 2160
ataaaatgag gaaattgcat cgcattgtct gagtaggtgt cattctattc tggggggtgg 2220

ggtggggcag gacagcaagg gggaggattg ggaagacaat agcaggcatg ctggggatgc 2280
ggtgggctct atggcttctg aggcaagctt 2310

<210> 4
<211> 1115
<212> DNA
<213> Artificial sequence

<220>
<223> Nucleotide sequence of the fourth nucleic acid fragment

<400> 4
aagcttgaat tcgccgcggg tgggggcgtt accatcccta catggaccca gttgtcgtat 60
aattttttcc ccccccccct tctccgcatg ggtgatgtcg ggtccaaact cccgacacca 120
ccagctggca tggtataaat caccggtgcg ccccccaaac catgtccggc agggggatgg 180
ggggcgaatg cggagggcac ccaacaacac cgggctaacc aggaaatccg tggccccggc 240
ccccaacaaa gatcgcggta gcccggccgt gtgacattat cgtccatacc gaccacaccg 300
acgaatcccc taagggggag gggccatttt acgaggagga ggggtataac aaagtctgtc 360
tttaaaaagc aggggttagg gagttgttcg gtcatatgct tcagtgcgaa cgaccaacta 420
ccccgatcat cagttatccg ccgccaccat gtggctgcag agcctgctgc tcttgggcac 480
tgtggcctgc agcatctctg cacccgcccg ctcgcccagc cccagcacgc agccctggga 540
gcatgtgaat gccatccagg aggcccggcg tctcctgaac ctgagtagag acactgctgc 600
tgagatgaat gaaacagtag aagtcatctc agaaatgttt gacctccagg agccgacctg 660
cctacagacc cgcctggagc tgtacaagca gggcctgcgg ggcagcctca ccaagctcaa 720
gggccccttg accatgatgg ccagccacta caagcagcac tgccctccaa ccccggaaac 780
ttcctgtgca acccagatta tcacctttga aagtttcaaa gagaacctga aggactttct 840
gcttgtcatc ccctttgact gctgggagcc agtccaggag tgagactgtg ccttctagtt 900
gccagccatc tgttgtttgc ccctcccccg tgccttcctt gaccctggaa ggtgccactc 960
ccactgtcct ttcctaataa aatgaggaaa ttgcatcgca ttgtctgagt aggtgtcatt 1020
ctattctggg gggtggggtg gggcaggaca gcaaggggga ggattgggaa gacaatagca 1080
ggcatgctgg ggatgcggtg ggctctatgg agctt 1115

<210> 5
<211> 144
<212> PRT
<213> Artificial sequence

<220>
<223> Amino acid sequence of GMCSF

<400> 5
Met Trp Leu Gln Ser Leu Leu Leu Leu Gly Thr Val Ala Cys Ser Ile
1               5                   10                  15
Ser Ala Pro Ala Arg Ser Pro Ser Pro Ser Thr Gln Pro Trp Glu His
            20                  25                  30
Val Asn Ala Ile Gln Glu Ala Arg Arg Leu Leu Asn Leu Ser Arg Asp
        35                  40                  45
Thr Ala Ala Glu Met Asn Glu Thr Val Glu Val Ile Ser Glu Met Phe
    50                  55                  60
Asp Leu Gln Glu Pro Thr Cys Leu Gln Thr Arg Leu Glu Leu Tyr Lys
65                  70                  75                  80
Gln Gly Leu Arg Gly Ser Leu Thr Lys Leu Lys Gly Pro Leu Thr Met
                85                  90                  95
Met Ala Ser His Tyr Lys Gln His Cys Pro Pro Thr Pro Glu Thr Ser
            100                 105                 110
Cys Ala Thr Gln Ile Ile Thr Phe Glu Ser Phe Lys Glu Asn Leu Lys
        115                 120                 125
Asp Phe Leu Leu Val Ile Pro Phe Asp Cys Trp Glu Pro Val Gln Glu
    130                 135                 140

## Description

### Cross-references to related applications

This application claims the priority of Chinese patent application filed with the Chinese Patent Office on July 16, 2019, with the application number 2019106445393, entitled "Virus and Tumor Therapeutic Drug for Specifically Killing Tumor cells", the entire content of which is incorporated by reference in this application.

### Technical Field

The present disclosure relates to the field of biotechnology, in particular, to virus and tumor therapeutic drug for specifically killing tumor cells.

### Background Art

Conquering cancer is a world-wide challenge. The current treatment of cancer mainly relies on traditional chemoradiotherapy combined with chimeric antigen receptor T cell immunotherapy (CAR-T therapy) and antibody therapy developed in recent years. However, each treatment modality either provides limited efficacy or produces severe side effects or potential safety risks. It is imperative to develop new, safe and effective treatment options. Studies have shown that the development of oncolytic viruses to treat cancer seems promising.

Oncolytic virus selectively replicates in tumor cells, thus lyzing the cells. Moreover, the cellular debris of the lyzed cells induces tumor specific immunity, which in return helps kill tumor cells *in situ* or attack metastasized tumor cells. Because of the unique features of oncolytic viruses, oncolytic viruses have been demonstrated to offer a promising cancer treatment strategy.

Oncolytic viruses are genetically engineered lytic viruses, which selectively replicate in and kill tumor cells. Up to date, a variety of strategies have been exploited for an oncolytic virus to achieve targeting. A widely used strategy for generating an oncolytic virus is to delete one or more non-essential genes from the viral genome, which encodes proteins to interfere with the antiviral pathway in normal cells. Because the anti-viral functions in tumor cells are either missing or greatly weakened, recombinant oncolytic viruses cannot propagate in normal cells but can replicate in tumor cells, thus achieving tumor selectivity. Several genes involved in the WNT signaling pathway including *RAS, TP53, RB1* and *PTEN,* interferons and several cytokines have been shown to play important roles in viral defenses in normal cells. However, those anti-viral mechanisms are abrogated or attenuated by viruses. Therefore, recombinant viruses generated by deleting viral genes which antagonize the antiviral functions of genes such as *RAS* and *P53,* interferons or antiviral-cytokines, would be safe to normal cells but can replicate in tumor cells. The first oncolytic virus Imlygic (T-vec) approved by the FDA was developed by deleting ICP34.5 and ICP47, two genes from type 1 herpes simplex virus (HSV-1). The former prevents protein synthesis from shut-down in normal cells leading to viral replication, and the latter inhibits the presentation of antigens to allow the virus to evade anti-viral immune response. Because the signaling pathway leading to the shut-down of protein synthesis, and antigen presentation are significantly impaired in tumor cells, T-vec can grow in tumor cells. Pexa-Vec, which is currently in phase 3 clinical trial, was generated by deleting thymidine kinase gene from vaccinia virus so that Pexa-Vec can only replicate in dividing cells with high kinase activity, such as tumor cells.

The clinical performance of currently available oncolytic viruses is generally poor, and clinical application is very limited. In order to increase the effectiveness and broad-spectrum of oncolytic viruses, the viral genome should be kept intact when an oncolytic virus is designed.

Based on the concept, this disclosure is hereby provided.

### Summary

The purpose of the present disclosure is to provide a virus and a tumor therapeutic drug, which specifically kills tumor cells while it is safe to non-tumor cells. Specifically, the virus genome is relatively complete and the virus can kill a variety of tumor cells.

The present disclosure is implemented as follows.

In the first aspect, the present disclosure provides a virus for specifically killing tumor cells. The virus is a recombinant oncolytic virus with an exogenous promoter inserted into the viral genome. The exogenous promoter is located upstream of an essential gene of the virus to drive the expression of an essential gene in tumor cells but not in normal cells.

The virus provided by the present disclosure is a recombinant oncolytic virus. An exogenous promoter is inserted upstream of an essential gene in the genome, and the exogenous promoter replaces the natural endogenous promoter to drive the expression of the essential gene in tumor cells, and the oncolytic virus can replicate normally and specifically kill tumor cells through its replication. In normal cells, the exogenous promoter is inactive, and cannot drive the expression of essential genes; therefore, the virus is safe for normal cells.

It should be noted that no viral genes in the recombinant oncolytic virus genome provided in the present disclosure have been deleted or destroyed. Therefore, the recombinant oncolytic virus provided in the present disclosure can replicate in and kill a variety of tumor cells effectively.

In embodiments of the present disclosure, the exogenous promoter is a tumor specific promoter, and the tumor cell-specific promoter can specifically drive the expression of the regulated essential genes in tumor cells, and the oncolytic virus can replicate robustly to specifically kill tumor cells. Meanwhile, in normal cells, the specific promoter of tumor cells is not active and will not drive the expression of essential genes; thus, the virus is safe for normal cells.

The tumor cell-specific promoter is selected from the group consisting of telomerase reverse transcriptase (*hTERT*) promoter, human epidermal growth factor receptor-2 (*HER-2*) promoter, *E2F1* promoter, *osteocalcin* promoter, *carcinoembryonic antigen* promoter, *survivin* promoter and *ceruloplasmin* promoter.

In some embodiments of the present disclosure, the virus also has an enhancer inserted into the viral genome, and the enhancer is located between the exogenous promoter and the essential gene to enhance the expression of the essential gene.

In some embodiments of the present disclosure, the enhancer is either a CMV or SV40 enhancer.

In some embodiments of the present disclosure, the regulated essential genes can be one or more than one, and the expression of each essential gene is controlled by an independent exogenous promoter and the enhancer.

In some embodiments of the present disclosure, an additional copy of the regulated essential gene is inserted into the genome. Such that, the regulated viral gene can be expressed sufficiently to support viral replication even in tumor cells in which the tumor specific promoter activity is low, thus expanding the application of the oncolytic virus for treatment of tumors.

In some embodiments of the present disclosure, an immunostimulatory factor expression sequence is inserted into the viral genome, in which a viral late gene promoter is used to drive the expression of the immunostimulatory factor expression sequence. Herein, the viral late gene promoter activity is controlled by the gene product of the regulated essential gene.

In some embodiments of the present disclosure, the immunostimulatory factor expressed from the immunostimulatory factor expression sequence is either interleukin 12 (IL-12) or granulocyte-macrophage colony stimulating factor (GMCSF).

In some embodiments of the present disclosure, the promoter used is the glycoprotein D promoter (gD promoter) for HSV-1 while the viral late gene is adenovirus late gene E3 promoter for adenovirus.

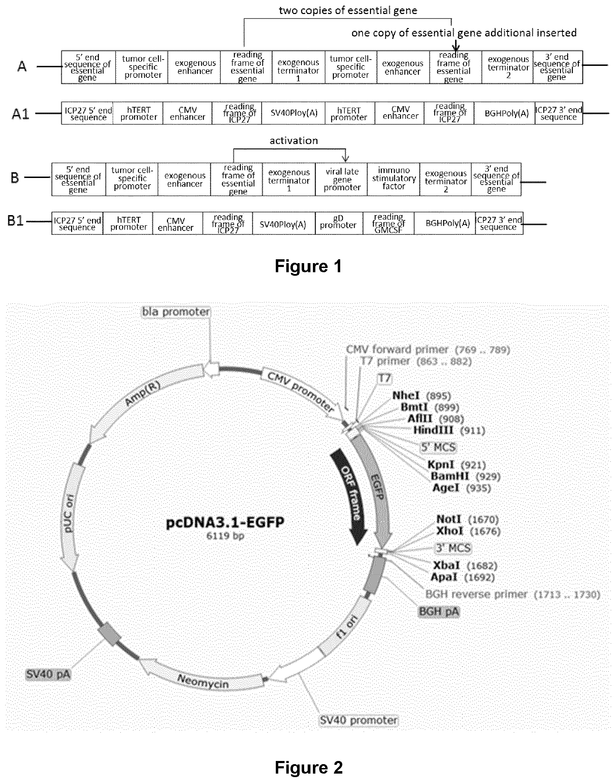

The viral late gene promoter activity is regulated by the gene product of the regulated essential gene, so that only when the regulated essential gene is expressed, the viral late gene promoter will be activated, thus driving the expression of downstream immunostimulatory factor (as shown in B of Figure 1). The regulated essential genes can only be expressed in tumor cells, thus resulting in the expression of immunostimulatory factor in tumor cells, which attract immune cells to infiltrate the tumor site and enhance anti-tumor immunity. In the case of entering normal cells by the oncolytic virus, immunostimulatory factors are not expressed, and thus do not increase safety risk.

In some embodiments of the present disclosure, the recombinant oncolytic virus is selected from the group consisting of HSV, coxsackie virus, influenza virus, vaccinia virus, measles virus, poliovirus, mumps virus, vesicular stomatitis virus, Newcastle disease virus and adenovirus.

It should be noted that several essential genes can be selected for regulation to enhance safety profile, for example:
when the recombinant oncolytic virus is HSV, one or more essential genes can be selected from the group consisting of envelope glycoprotein L, uracil DNA glycosylase, capsid protein, helicase proenzyme subunit, DNA replication initiation binding unwindase, derived protein of myristic acid, deoxyribonuclease, coat serine/threonine protein kinase, DNA packaging terminase subunit 1, coat protein UL16, DNA packaging protein UL17, capsid triplex subunit 2, major capsid protein, envelope protein UL20, nucleoprotein UL24, DNA packaging protein UL25, capsid mature protease, capsid protein, envelope glycoprotein B, single-stranded DNA-binding protein, DNA polymerase catalytic subunit, nuclear egress layer protein, DNA packaging protein UL32, DNA packaging protein UL33, nuclear egress membrane protein, large capsid protein, capsid triplex subunit 1, ribonucleotide reductase subunit 1, ribonucleotide reductase subunit 2, capsule host shutoff protein, DNA polymerase processing subunit, membrane protein UL45, coat protein VP13/14, trans-activating protein VP16, coat protein VP22, envelope glycoprotein N, coat protein UL51, unwindase-primase primase subunit, envelope glycoprotein K, regulatory protein ICP27, nucleoprotein UL55, nucleoprotein UL56, transcription regulation factor ICP4, regulatory protein ICP22, envelope glycoprotein D, and membrane protein US8A.

When the recombinant oncolytic virus is adenovirus, one or more essential genes can be selected from the group consisting of early protein 1A, early protein 1B 19K, early protein 1B 55K, encapsidation protein Iva2, DNA polymerase, terminal protein precursor pTP, encapsidation protein 52K, capsid protein precursor pIIIa, pentomer matrix, core protein pVII, core protein precursor pX, core protein precursor pVI, hexonmer, proteinase, single-stranded DNA-binding protein, hexamer assembly protein 100K, protein 33K, encapsidation protein 22K, capsid protein precursor, protein U, fibrin, open reading frame 6/7 of regulatory protein E4, regulatory protein E4 34K, open reading frame 4 of regulatory protein E4, open reading frame 3 of regulatory protein E4, open reading frame 2 of regulatory protein E4, and open reading frame 1 of regulatory protein E4.

When the recombinant oncolytic virus is vaccinia virus, one or more essential genes can be selected from the group consisting of nucleotide reductase small-subunit, serine/threonine kinase, DNA-binding viral core protein, polymerase large-subunit, RNA polymerase subunit, DNA polymerase, sulfhydryl oxidase, hypothetical DNA-binding viral nucleoprotein, DNA-binding phosphoprotein, nucleoid cysteine proteinase, RNA helicase NPH-II, hypothetical metalloproteinase, transcription elongation factor, glutathione-type protein, RNA polymerase, hypothetical viral nucleoprotein, late transcription factor VLTF-1, DNA-binding viral nucleoprotein, viral capsid protein, polymerase small-subunit, RNA polymerase subunit rpo22 depending on DNA, RNA polymerase subunit rpo147 depending on DNA, serine/threonine protein phosphatase, IMV heparin-binding surface protein, DNA-dependent RNA polymerase, late transcription factor VLTF-4, DNA topoisomerase type I, mRNA capping enzyme large-subunit, viral core protein 107, viral core protein 108, uracil-DNA glycosylase, triphosphatase, 70kDa small subunit of early gene transcription factor VETF, RNA polymerase subunit rpo18 depending on DNA, nucleoside triphosphate hydrolase-I, mRNA capping enzyme small-subunit, rifampicin target site, late transcription factor VLTF-2, late transcription factor VLTF-3, disulfide bond forming pathway, precursor p4b of core protein 4b, core protein 39kDa, RNA polymerase subunit rpo19 depending on DNA, 82kDa large subunit of early gene transcription factor VETF, 32kDa small subunit of transcription factor VITF-3, IMV membrane protein 128, precursor P4a of core protein 4a, IMV membrane protein 131, phosphorylated IMV membrane protein, IMV membrane protein A17L, DNA unwindase, viral DNA polymerase processing factor, IMV membrane protein A21L, palmitoyl protein, 45kDa large subunit of intermediate gene transcription factor VITF-3, RNA polymerase subunit rpo132 depending on DNA, RNA polymerase rpo35 depending on DNA, IMV protein A30L, hypothetical ATP enzyme, serine/threonine kinase, EEV mature protein, palmitoylated EEV membrane glycoprotein, IMV surface protein A27L, EEV membrane phosphate glycoprotein, IEV and EEV membrane glycoproteins, EEV membrane glycoprotein, disulfide bond forming pathway protein, hypothetical viral nucleoprotein, IMV membrane protein I2L, poxvirus myristoyl protein, IMV membrane protein L1R, late 16kDa hypothetical membrane protein, hypothetical virus membrane protein H2R, IMV membrane protein A21L, chemokine-binding protein, epidermal growth factor-like protein, and IL-18 binding protein.

When the recombinant oncolytic virus is coxsackie virus, one or more essential genes can be selected from the group consisting of protein Vpg, core protein 2A, protein 2B, RNA unwindase 2C, protein 3A, proteinase 3C, reverse transcriptase 3D, coat protein Vp4, and protein Vp1.

When the recombinant oncolytic virus is measles virus, one or more essential genes can be selected from the group consisting of nucleoprotein N, phosphoprotein P, matrix protein M, transmembrane glycoprotein F, transmembrane glycoprotein H, and RNA-dependent RNA polymerase L.

When the recombinant oncolytic virus is mumps virus, one or more essential genes can be selected from the group consisting of nucleoprotein N, phosphoprotein P, fusion protein F, and RNA polymerase L.

When the recombinant oncolytic virus is vesicular stomatitis virus, one or more essential genes can be selected is one or more selected from the group consisting of glycoprotein G, nucleoprotein N, phosphoprotein P and RNA polymerase L.

When the recombinant oncolytic virus is poliovirus, one or more essential genes can be selected from the group consisting of capsid protein VP1, capsid protein VP2, capsid protein VP3, cysteine protease 2A, protein 2B, protein 2C, protein 3A, protein 3B, proteinase 3C, protein 3D, and RNA-directed RNA polymerase.

When the recombinant oncolytic virus is influenza virus, one or more essential genes can be selected from the group consisting of hemagglutinin, neuraminidase, nucleoprotein, membrane protein M1, membrane protein M2, polymerase PA, polymerase PB1-F2, and polymerase PB2.

In some embodiments of the present disclosure, when the recombinant oncolytic virus is HSV-1, the essential gene is ICP27, and the viral late gene promoter is the gD promoter, which is regulated by the ICP27 gene product.

In some embodiments of the present disclosure, when the recombinant oncolytic virus is adenovirus, the essential gene is E1A, and the viral late gene promoter is the late gene E3 promoter, and E1A gene expression can activate the E3 gene promoter.

In the second aspect, the present disclosure provides a tumor therapeutic drug, which contains the virus that specifically kills tumor cells as described above.

In some embodiments of the present disclosure, the drug further contains a pharmaceutically acceptable carrier.

It should be understood that "pharmaceutically acceptable carrier" refers to one or more compatible solid or liquid fillers or gel substances, which are suitable for use in humans, and must be of high purity and low toxicity. "Compatibility" here refers to the ability of each component of the drug to mix with the virus that specifically kills tumor cells of the present disclosure and other components of the drug, without significantly affecting the therapeutic effect.

In the third aspect, the present disclosure provides an isolated nucleic acid sequence for preparing the aforementioned virus, wherein the nucleic acid sequence contains a core sequence for inserting into a target site of a target virus, and the core sequence includes a promoter. The target site is located upstream of the essential gene of the target virus, and the promoter is used to drive the expression of the regulated essential gene in tumor cells but not in normal cells.

In the above, the target virus is a wild-type oncolytic virus, such as wild-type HSV, coxsackie virus, influenza virus, vaccinia virus, measles virus, poliovirus, mumps virus, vesicular stomatitis virus, Newcastle disease virus or adenovirus, or the like.

In some embodiments of the present disclosure, the promoter is a tumor cell-specific promoter, which can specifically drive the expression of downstream essential genes in tumor cells, and the oncolytic virus can replicate robustly, which specifically kills tumor cells through its replication; when in normal cells, the tumor cell-specific promoter does not drive the expression of essential genes, and thereby the virus does not present safety issue to normal cells.

In embodiments of the present disclosure, the tumor cell -specific promoter is selected from the group consisting of *hTERT* promoter, *HER-2* promoter, *E2F1* promoter, *osteocalcin* promoter, *carcinoembryonic antigen* promoter, *survivin* promoter and *ceruloplasmin* promoter.

In some embodiments of the present disclosure, the core sequence further includes an enhancer, which is located downstream of the promoter and upstream of the essential gene, that is, the enhancer is located between the promoter and the essential gene to enhance the expression of the essential gene.

In some embodiments of the present disclosure, the enhancer is either CMV enhancer or SV40 enhancer.

In some embodiments of the present disclosure, the core sequence further includes an immunostimulatory factor expression sequence and a viral late gene promoter that drives the expression of the immunostimulatory factor from the expression sequence. Herein, the viral late gene promoter is regulated by the gene product of the essential gene.

In some embodiments of the present disclosure, the immunostimulatory factor expressed from the immunostimulatory factor expression sequence is either interleukin 12 (IL-12) or granulocyte-macrophage colony stimulating factor (GMCSF).

In some embodiments of the present disclosure, when the virus is HSV-1, the essential gene is ICP27, and the viral late gene promoter is the gD promoter.

Alternatively, when the virus is an adenovirus, the essential gene is E1A, and the viral late gene promoter is the adenovirus late gene E3 promoter.

In some embodiments of the present disclosure, the nucleic acid molecule contains 5' and 3' arms, which are homologous to the sequences upstream and downstream the target site. The 5' arm does not contain the endogenous promoter of the regulated essential gene.

The present disclosure also provides a vector, which contains the forementioned nucleic acid sequence.

### Brief Description of Drawings

Exemplary features of the present disclosure, its nature and various advantages will be apparent from the accompanying drawings and the following detailed description of various embodiments. Non-limiting and non-exhaustive embodiments are described with reference to the accompanying drawings. The sizes and relative positions of elements in the drawings are not necessarily drawn to scale. For example, the shapes of various elements are selected, enlarged, and positioned to improve drawing legibility. The particular shapes of the elements as drawn have been selected for ease of recognition in the drawings. One or more embodiments are described hereinafter with reference to the accompanying drawings in which
Figure 1 Schematic showing of the genome structure of the recombinant oncolytic virus provided by the examples of the present disclosure. A: Schematic showing of the genome structure of the recombinant oncolytic virus 1 of Example 1, wherein an additional copy of a regulated essential gene is inserted. The regulatory element contains an exogenous tumor cell-specific promoter and an enhancer located upstream of the reading frame of each copy of the regulated essential gene. A1 Schematic showing of the genome structure of the recombinant oncolytic virus 1 (oHSV-BJTT), wherein the virus is HSV-1, the essential gene was ICP27, tumor -specific promoter was *hTERT,* and the enhancer is the CMV enhancer. B Schematic showing of the genome structure of the recombinant oncolytic virus 2 provided by Example 2, the first expression cassette contains tumor specific promoter, an enhancer, the open reading frame of the regulated essential gene, followed by a ploy (A) sequence. and the second expression cassette contains a viral late gene promoter, and the open reading frame of an immunostimulatory factor followed by a ploy (A) sequence. B1 Schematic showing of the genome structure of the recombinant oncolytic virus 2 (oHSV-BJGMCSF), wherein the virus is HSV-1, the essential gene is HSV-1 ICP27, the tumor cell-specific promoter is *hTERT,* the enhancer is the CMV enhancer, the viral late gene promoter is HSV-1 gD promoter, and the immunostimulatory factor was GM-CSF.
Figure 2 Schematic showing of the parental plasmid pcDNA3.1-EGFP unitized for constructing a plasmid expressing HSV-1 ICP27. In the plasmid, EGFP is constitutively expressed under the control of the CMV promoter and the plasmid contains neomycin-resistant gene expression sequence.

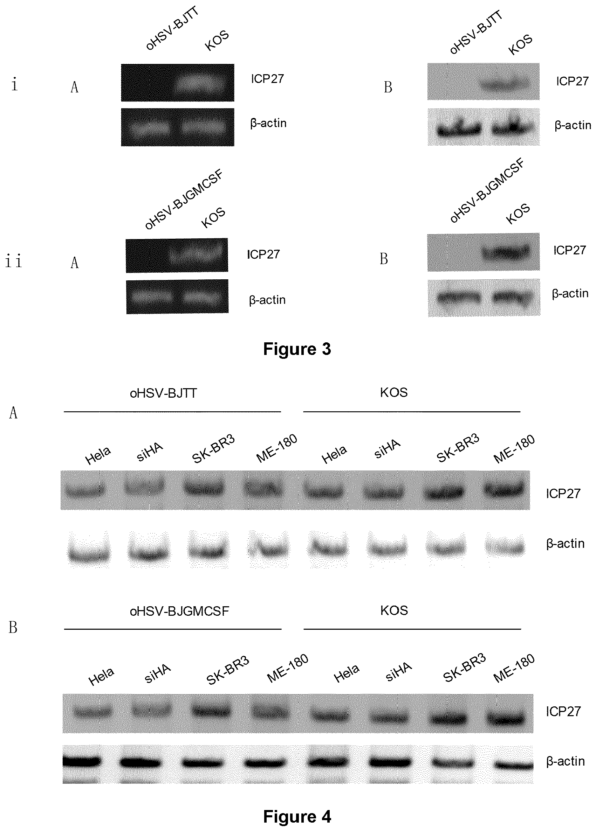

Figure 3 The expression of ICP27 from the recombinant virus oHSV-BJTT or oHSV-BJGMCSF in African green monkey kidney cells (Vero) (normal cells) is lower than the detection limit. Vero cells were infected with 3 multiplicities of infection (MOIs, virus/cell) oHSV-BJTT, oHSV-BJGMCSF or HSV-1 wild-type virus KOS. One day later, the cells were harvested, RNA was isolated, and protein extracted, and ICP27 mRNA (A) was detected by reverse transcription combined with semi-quantitative PCR, and HSV-1 ICP27 protein (B) was detected by Western blotting assays. (i): oHSV-BJTT; and (ii): oHSV-BJGMCSF.
Figure 4 No significant difference was observed in ICP27 protein expression of in tumor cells from the recombinant virus oHSV-BJTT, oHSV-BJGMCSF or wild-type virus KOS. The tumor cells Hela, siHA, SK-BR3 and ME-180 were respectively infected with 3 MOI oHSV-BJTT, oHSV-BJGMCSF or wild-type virus KOS. One day later, the cells were collected, the protein was extracted, and the ICP27 protein was detected by Western blotting assays. A: oHSV-BJTT; B: oHSV-BJGMCSF.

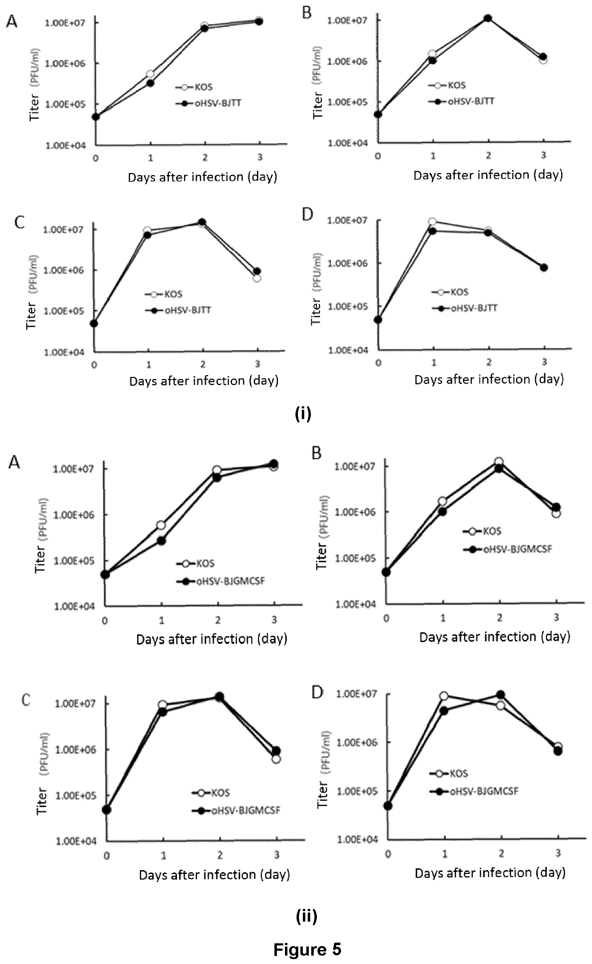

Figure 5 Basically identical replication kinetics was observed among oncolytic viruses oHSV-BJTT, oHSV-BJGMCSF and wild-type virus KOS. Various tumor cells were infected with 0.1 MOI oHSV-BJTT, oHSV-BJGMCSF or KOS. At different days after infection, the cells and culture medium were collected, and the virus remaining in the cells was released into the culture medium through three freeze-thawing cycles. Complementing cells were infected with the virus, and virus titer (plaque forming unit/ml, PFU/ml) was determined by the plaque method. (i): oHSV-BJTT; and (ii): oHSV-BJGMCSF. In (i) and (ii): A: cervical tumor Hela cell; B: cervical squamous cancer siHa cell; C: breast cancer SK-BR3 cell; D: breast cancer ME-180 cell.

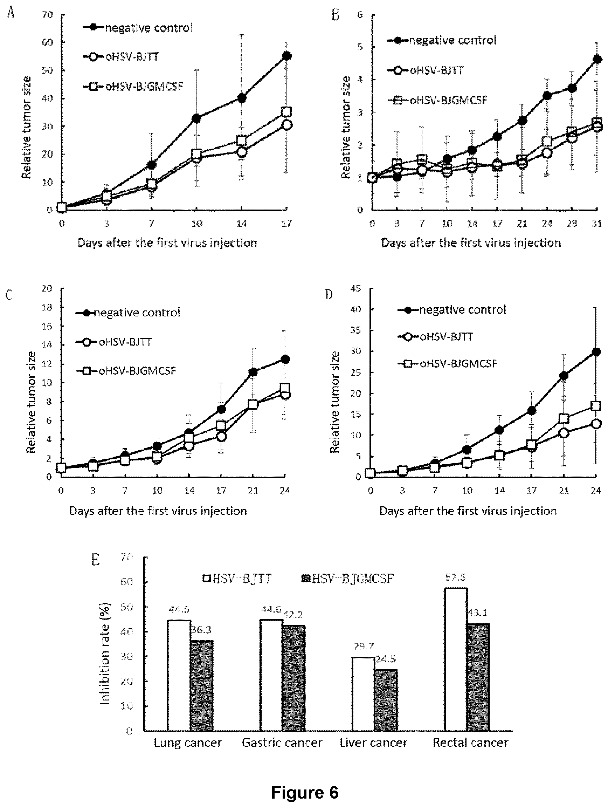

Figure 6 Recombinant oncolytic viruses oHSV-BJTT and oHSV-BJGMCSF significantly inhibit the proliferation of lung cancer, gastric cancer, liver cancer and rectal cancer in animal tumor models by recombinant oncolytic viruses oHSV-BJTT and oHSV-BJGMCSF. A tumor animal model was established. After the tumor grew to 50-80 mm³, the oncolytic virus was injected into the tumor every 3 days for a total of 3 times. PBS (without oncolytic virus) was injected as a negative control. After the oncolytic virus was injected, the tumor volume was measured twice a week. When the negative control animals needed to be euthanized, the experiment ended. A tumor growth curve was plotted based on the tumor volume (in the figure, A: lung cancer; B: gastric cancer; C: liver cancer; D: rectal cancer), and the relative inhibition rate (E) was calculated by comparing the tumor volume in the test group at the end of the test with that observed in the negative control.

### Detailed Description of the Embodiments

In order to demonstrate the features of the present disclosure, its nature and various advantages, exemplary embodiments were executed and are described in details below. All experiments were conducted using standard methods as described in literature. Reagents were purchased from commercial providers and used according to the instructions of the manufacturers.

The features and performance of the oncolytic virus provided in the present disclosure will be further described in detail below in conjunction with examples.

### Example 1

The recombinant oncolytic virus 1 provided in this example was constructed with wild-type HSV type 1 KOS as the starting material. The genome of the recombinant oncolytic virus 1 has the following structural features.

Referring to A in Figure 1, in the genome of the recombinant oncolytic virus 1 of this example, an additional copy of an essential gene is inserted so that the number of copies of the essential gene was two, and the regulatory element is composed of a tumor cell-specific promoter and an enhancer is inserted into upstream of the reading frame of each copy of the essential gene to replace the endogenous promoter of the essential gene, thereby driving the expression of the essential gene in tumor cells. There is an exogenous terminator 1 downstream of the reading frame of the first copy of the essential gene, and an exogenous terminator 2 downstream of the reading frame of the second copy of the essential gene, and the essential gene is driven by the above-mentioned regulatory element to be specifically expressed in tumor cells.

Referring to A1 in Figure 1, specifically, in this example, the regulated essential gene is ICP27, and an additional copy of ICP27 is inserted. The tumor cell-specific promoter is hTERT, the enhancer is the CMV enhancer, the exogenous terminator 1 is SV40 Poly (A), and the exogenous terminator 2 is BGH Poly (A).

In the following text, the virus is named as oHSV-BJTT.

### Example 2

The recombinant oncolytic virus 2 provided in this example contains a regulated essential gene and a late viral promoter-driven immunostimulatory factor expression cassette. The genome of the recombinant oncolytic virus 2 possesses the following structural features.

Referring to B in Figure 1, a first regulatory element is composed of a tumor -specific promoter and an enhancer is located upstream of the reading frame of the regulatory essential gene in the genome of the recombinant oncolytic virus 2 of this example; a second regulatory element set composed of a viral late gene promoter and an immunostimulatory factor reading frame is located downstream of the reading frame of the essential gene. Herein, the viral late gene promoter is regulated by essential gene expression product; there are an exogenous terminator 1 located downstream of essential gene reading frame and an exogenous terminator 2 located downstream of the reading frame of the immunostimulatory factor.

Referring to B1 in Figure 1, specifically, in this example, the essential gene is ICP27, the tumor cell-specific promoter is hTERT, the enhancer is the CMV enhancer, and the exogenous terminator 1 is SV40 Poly(A), the viral late gene promoter is the gD promoter, the immunostimulatory factor reading frame is the GMCSF reading frame, and the exogenous terminator 2 is BGH Ploy(A).

In the following text, the recombinant oncolytic virus is named as oHSV-BJGMCSF.

### Example 3

This example provides the methods for preparing the recombinant oncolytic virus provided in the foregoing Example 1 or 2, and the specific manipulations were as follows.
(1) Preparation of complementing cells expressing HSV-1 ICP27
   (a) plasmid construction: Using the DNA of wild-type HSV-1 KOS as a template, the encoding region of ICP27 was amplified by PCR, and inserted into *Hind*III and X*ba*I sites of plasmid pcDNA3.1-EGFP expressing the neomycin-resistant gene (see Figure 2 for the structure) to replace EGFP. The recombinant plasmid was named as ICP27 expression plasmid, and the ICP27 gene was expressed under the control of the CMV promoter.
   (b) G418 dose determination for selection: Vero cells were treated with G418 of different concentrations, the culture medium containing G418 was refreshed every three days with media containing G418 of different concentrations, and cell death was monitored every day. The minimal concentration of G418 required for all cells to die after 6 days of G418 treatment was determined. Such a concentration of G418 (500 µg/ml) was utilized for complementing cell establishment.
   (c) Cell line establishment: Totally 3.5×10⁵ Vero cells were seeded into each well of a 6-well cell culture plate and cultured overnight in an antibiotic-free culture medium, and 4 µg of the ICP27 expression plasmid obtained in step (a) were transfected into cells in each well with Lipofectamine 2000. After 24 hours of culture, cells in each well were harvested, and diluted by 1:20, 1:40, and 1:60, respectively. Cells were cultured in the culture medium containing G418, and the medium was refreshed with medium containing G418 every 3 days. After 6-7 changes of the culture medium, the clones were collected and propagated step by step from the 24-well plate to T150 tissue culture flasks. Subsequently, the protein was separated and the expression of ICP27 detected by Western blotting assays. The cells with the highest level of ICP27 expression were selected as the complementing cells to support the growth and replication of replication-defective viruses in which ICP27 was not expressed; the cells were named as C_{ICP27}
   The complementing cell has been preserved at China Center for Type Culture Collection (CCTCC), Wuhan University, Luojiashan, Wuchang, Wuhan City on April 24, 2019 with a preservation number of CCTCC NO. C201974.
(2) Preparation of the parental virus rHSV-EGFP
   The wild-type HSV type 1 was used as the starting material, the recombinant parental virus HSV-EGFP was obtained by homologous recombination between plasmid and KOS genome. In HSV-EGFP, HSV-ICP27 was replaced by EGFP. HSV-EGFP will serve as the parental virus for generating the oncolytic viruses provided in this disclosure. The manipulations are detailed as follows:
   (a) the first nucleic acid fragment was synthesized and its nucleotide sequence is shown in SEQ ID NO. 1:
      From 5' to 3' end, the fragment includes the following elements: ICP27 5' sequence, CMV promoter, Kozak sequence, EGFP encoding frame, BGH Poly(A) and ICP27 3' sequence.
      Sequence seen in SEQ ID NO. 1:
      site 1-6: irrelevant sequence, increasing the end length to facilitate enzyme digestion;
      site 7-12: *Xho1* site, C/TCGAG;
      site 13-575: ICP27 5' end sequence;
      site 576-1163: CMV promoter;
      site 1164-1174: interval sequence;
      site 1175-1180: Kozak sequence, increasing protein expression;
      site 1181-1900: EGFP encoding frame;
      site 1901-2145: BGH Poly(A);
      site 2146-2667: ICP27 3' end sequence;
      site 2668-2673: *Hind*III site, A/AGCTT; and
      site 2674-2679: irrelevant sequence, increasing the end length to facilitate enzyme digestion.
   (b) the first fragment was cleaved and ligated to the *Hind*III and *Xho1* sites of the pcDNA3.1-EGFP plasmid, and the resulting recombinant plasmid was named as EGFP expression plasmid.
   (c) 3.5×10⁵ above-mentioned complementing C_{ICP27} cells were seeded into each well in 6-well cell culture plate and cultured overnight in an antibiotic-free culture medium.
   (d) the cells were infected with wild-type virus KOS of 0.1, 0.5, 1, 3 MOI (virus/cell), respectively. After 1 hour, the above-mentioned EGFP expression plasmid (4 µg DNA/well) was transfected into the cells using Lipofectamine 2000. Four hours later, the transfection mixture was replaced with complete medium. After all the cells became spherical, the cells and culture medium were collected, and after three cycles of freeze-thawing, the mixture was centrifuged and the supernatant collected by centrifugation, diluted, and infected by the above-mentioned complementing cell C_{ICP27}. The viruses were isolated by using plaque separation method. Four to 5 days later, the green virus plaque was selected under a fluorescence microscope, and then the obtained virus plaques were subjected to 2 or 3 rounds of screening to obtain pure virus plaques, and the virus was propagated. The recombinant virus with ICP27 replaced by EGFP was named as HSV-EGFP. HSV-EGFP served as the parental virus for generation of the oncolytic viruses provided in this disclosure
(3) Construction of recombinant plasmid
   (a) the TA cloning plasmid was modified such that the multiple cloning site in the plasmid only contains a X*hol* site. The resulting plasmid was named as TA-Xhol plasmid, for subsequent use.
   (b) the second nucleic acid fragment was synthesized, and its nucleotide sequence was shown in SEQ ID NO. 2.

The nucleic acid fragment from 5' to 3' end contains the following elements: ICP27 5' end sequence (excluding the natural promoter), *hTERT* promoter, CMV enhancer sequence, ICP27 open reading frame, SV40 Poly(A) sequence and ICP27 3' end sequence. In addition, the 5' and 3' ends of the second nucleic acid fragment each contain one *Xhol* site, and there is one *Hind*III site between the SV40 Poly(A) and ICP27 3' end sequence.

In SEQ ID NO. 2:
site 1-6: *Xho*1 site;
site 7-517: ICP27 5' end non-coding region;
site 518-973: *hTERT* promoter;
site 974-1039: CMV enhancer;
site 1040-2578: ICP27 open reading frame;
site 2579-3050: SV40 Poly(A);
site 3051-3056: *Hind*III site;
site 3057-3576: ICP27 3' end non-coding region; and
site 3577-3582: *Xho1* site.
   (c) the second nucleic acid fragment was cleaved and inserted into the *Xhol* site of the plasmid TA-Xhol, and the resulting recombinant plasmid was named as: TA-Xhol-hTERT-CMVminimal-ICP27 plasmid, for subsequent use.
   (d) the third nucleic acid fragment was artificially synthesized, and its nucleotide sequence is shown in SEQ ID NO. 3:

The nucleic acid fragment from 5' to 3' end contains the following elements: *hTERT* promoter plus CMV enhancer sequence, ICP27 open reading frame and BGH Poly(A) sequence; The 5' and 3' ends each contain one *Hind*III site.

In SEQ ID NO. 3:
site 1-6: *Hind*III site;
site 7-462: *hTERT* promoter;
site 463-528: CMV enhancer;
site 529-2067: ICP27 open reading frame;
site 2068-2304: BGH Poly(A); and
site 2305-2310: *Hind*III site.
   (e) the fourth nucleic acid fragment was artificially synthesized, and its nucleotide sequence is shown in SEQ ID NO. 4:

The nucleic acid fragment from 5' to 3' end contains the following elements: gD promoter, Kozak sequence, GMCSF open reading frame and BGH Poly(A) sequence; each of the 5' and 3' ends contains one *Hind*III site.

In SEQ ID NO. 4:
site 1-6: *Hind*III site;
site 7-439: gD promoter;
site 440-448: Kozak sequence;
site 449-883: GMCSF open reading frame;
site 884-1100: BGH Poly(A); and
site 1100-1115: *Hind*III site.
Herein, the amino acid sequence (SEQ ID NO. 5) of GMCSF is MWLQSLLLLGTVACSISAPARSPSPSTQPWEHVNAIQEARRLLNLSRDTAAE MNETVEVISEMFDLQEPTCLQTRLELYKQGLRGSLTKLKGPLTMMASHYKQ HCPPTPETSCATQIITFESFKENLKDFLLVIPFDCWEPVQE.
(f) the third nucleic acid fragment was digested with *Hind*III and inserted into the *Hind*III site of the TA-Xhol-hTERT-CMVminimal-ICP27 plasmid. The resulting recombinant plasmid was named as ICP27-TT.

The fourth nucleic acid fragment was digested with *Hind*III and inserted into the *Hind*III site of the TA-Xhol-hTERT-CMVminimal-ICP27 plasmid. The resulting recombinant plasmid was named as ICP27-GMCSF.

### (4) Construction of recombinant oncolytic viruses oHSV-BJTT and oHSV-BJGMCSF

(a) the above-mentioned complementing C_{ICP27} cells were seeded into a 6-well cell culture plate with 3.5×10⁵ cells per well, and cultured overnight in an antibiotic-free culture medium.
(b) the complementing C_{ICP27} cells were respectively infected at an MOI of 0.1, 0.5, 1, and 3 of the parental virus HSV-EGFP; 1 hour later, plasmid ICP27-TT DNA (4µg DNA/well) was transfected to cells by using Lipofectamine 2000. After 4 hours, the transduction solution was replaced with complete medium. After all the cells were infected, the cells and culture solution were collected, through three times of freeze-thawing, the supernatant was collected by centrifugation, diluted, and infected by the above-mentioned complementing C_{ICP27} cells.
(c) the viruses were separated using plaque separation method. Four to 5 days later, virus plaques without green fluorescence were selected under a fluorescence microscope, and then the obtained virus plaques were subjected to 2 or 3 rounds of screening to obtain pure viruses, which were then propagated. DNA was isolated from the infected cells, and was amplified by PCR using specific primers and confirmed by sequencing. The recombinant oncolytic virus obtained was named as oHSV-BJTT.
(d) Repeated steps from a to d to obtain oncolytic oHSV-BJGMCSF.

These two viruses have been both preserved in the Chinese Center of Type Culture Collection (CCTCC), Wuhan University, Luojiashan, Wuchang, Wuhan on April 24, 2019. The preservation number of the oHSV-BJTT virus is CCTCC NO: V201922, and the preservation number of the oHSV-BJGMCSF virus is CCTCC NO: V201921.

### Experimental example 1

Detection of the expression of ICP27 in normal cells infected with recombinant oncolytic virus

Detection method: Vero cells were infected with HSV-1 wild-type virus KOS, oHSV-BJTT or oHSV-BJGMCSF at 3 MOI (virus/cell). One day later, the cells were collected, and RNA and protein were isolated. The expression level of ICP27 mRNA was detected using semi-quantitative PCR, and the expression level of HSV-1 ICP27 protein detected by Western blotting assays. Both mRNA and protein analysis used β-actin as loading control.

In the cells infected with KOS, ICP27 mRNA and protein were expressed to an easily detectable level. In the cells infected with oHSV-BJTT or oHSV-BJGMCSF, ICP27 mRNA and protein were below detectable level (Figure 3: A: mRNA; B: protein). It indicates that in normal cells, the expression of ICP27 mRNA and protein were expressed at a very low level or not expressed from oHSV-BJTT or oHSV-BJGMCSF.

### Experimental example 2

Detection of the expression of GMCSF and ICP27 in tumor cells infected with recombinant oncolytic virus

Detection method: 3 MOI oHSV-BJTT, oHSV-BJGMCSF or wild-type virus KOS was used to infect tumor cells, respectively: cervical tumor cells Hela, cervical squamous tumor cells siHA, breast tumor cells SK-BR3 and breast tumor cells ME-180. After 6 hours, a small portion of infected cell culture medium was collected. The content of GMCSF in the cell culture medium was analyzed by ELISA. One day later, all infected cells were collected, proteins isolated, and the ICP27 protein was detected by Western blotting assays. For protein analysis, β-actin was used as the loading control.

The GMCSF content in oHSV-BJGMCSF-infected cell culture medium reached a detectable level, but in oHSV-BJTT- or KOS-infected cell culture medium, the GMCSF was not detectable (Table 1). In different cells infected with oHSV-BJTT and oHSV-BJGMCSF, ICP27 protein level in one cell type is different from that observed in another cell type, but for a given cell type, there was not much difference in the levels of ICP27 protein in the cells infected by oHSV-BJTT, oHSV-BJGMCSF or by KOS (Figure 4). GMCSF can be expressed from oHSV-BJGMCSF in tumor cells; meanwhile, ICP27 can be expressed specifically from oHSV-BJTT and oHSV-BJGMCSF, and the expression level was not much different from that of wild-type virus.

**Table 1. GMCSF content in different tumor cell culture media with cells infected by oHSV-BJGMCSF (ng/ml)**

| Tumor cell type | Hela | siHA | BR-SK3 | ME-180 |
|---|---|---|---|---|
| oHSV-BJGMCSF | 4.5 | 5.6 | 9.5 | 7.2 |
| oHSV-BJTT | 0 | 0 | 0 | 0 |
| KOS | 0 | 0 | 0 | 0 |

### Experimental example 3

Detection of the replication kinetics of recombinant oncolytic viruses oHSV-BJTT and oHSV-BJGMCSF in tumor cells.

Detection method: Tumor cells were infected with 0.1 MOI of oHSV-BJTT, oHSV-BJGMCSF or KOS. After different days, the cells and culture medium were collected, separately, and the viruses remaining in the cells were released into the culture medium through three -80/37°C freeze-thawing cycles. The complementing C_{ICP27} cells were infected with the virus, and the virus titer (plaque forming unit/ml, PFU/ml) was determined by the plaque assay.

oHSV-BJTT (Figure 5i) and oHSV-BJGMCSF (Figure 5.ii) like KOS can replicate in all the four tumor cell types tested. For a given day after infection, the replication ability of oHSV-BJTT or oHSV-BJGMCSF was slightly lower than that of KOS (A: Hela cells; B: siHA cells; C: SK-BR3 cells and D: ME-180 cells), but there was no significant difference between oHSV-BJTT and KOS or between oHSV-BJGMCSF and KOS. The results demonstrates that genetic engineering of the virus whereby the oncolytic viruses are generated basically maintains the replicative ability of the virus.

### Experimental example 4

Detection of the ability of recombinant oncolytic viruses oHSV-BJTT and oHSV-BJGMCSF to kill tumor cells.

Detection method: Tumor cells including Hela cells, siHA cells, SK-BR3 cells and ME-180 cells, were respectively infected with 0.25 or 0.5 MOI oHSV-BJTT, oHSV-BJGMCSF or wild-type KOS. Cell survival rate was analyzed at different days after infection. The results are shown in Table 2-5 (for the recombinant oncolytic virus oHSV-BJTT) and Table 6-9 (for the recombinant oncolytic virus oHSV-BJGMCSF).

**Table 2. Survival rate (%) of Hela cells infected with oHSV-BJTT at different days after infection**

| Virus (MOI 0.25) | oHSV-BJTT | KOS |
|---|---|---|
| the first day | 56±3.0 | 47±2.8 |
| the second day | 37±1.5 | 25±1.3 |
| the third day | 15±0.8 | 8±0.5 |
| the fourth day | 5±0.2 | 0 |

| Virus (MOI 0.5) | oHSV-BJTT | KOS |
|---|---|---|
| the first day | 49±3.0 | 36±1.9 |
| the second day | 32±1.8 | 19±1.4 |
| the third day | 8±0.5 | 4±0.2 |
| the fourth day | 0 | 0 |

**Table 3. Survival rate (%) of siHA cells infected with oHSV-BJTT at different days after infection**

| Virus (MOI 0.25) | oHSV-BJTT | KOS |
|---|---|---|
| the first day | 25±2 | 15±0.9 |
| the second day | 5±0.5 | 0 |
| the third day | 0 | 0 |

| Virus (MOI 0.5) | oHSV-BJTT | KOS |
|---|---|---|
| the first day | 11±0.4 | 5±0.4 |
| the second day | 1±0.6 | 0 |
| the third day | 0 | 0 |

**Table 4. Survival rate (%) of SK-BR3 cells infected with oHSV-BJTT at different days after infection**

| Virus (MOI 0.25) | oHSV-BJTT | KOS |
|---|---|---|
| the first day | 25±3 | 10±0.8 |
| the second day | 0 | 0 |
| the third day | 0 | 0 |

| Virus (MOI 0.5) | oHSV-BJTT | KOS |
|---|---|---|
| the first day | 6±0.5 | 4±0.3 |
| the second day | 1±0.6 | 0 |
| the third day | 0 | 0 |

**Table 5. Survival rate (%) of ME-180 cells infected with oHSV-BJTT at different days after infection**

| Virus (MOI 0.25) | oHSV-BJTT | KOS |
|---|---|---|
| the first day | 18±1 | 9±0.5 |
| the second day | 7.4±0.4 | 0 |
| the third day | 0 | 0 |

| Virus (MOI 0.5) | oHSV-BJTT | KOS |
|---|---|---|
| the first day | 9±0.6 | 7±0.4 |
| the second day | 1±0.6 | 0 |
| the third day | 0 | 0 |

From Table 2-5, it can be seen that oHSV-BJTT can kill different cell types, and the ability is similar to that observed for KOS.

**Table 6. Survival rate (%) of Hela cell infected with oHSV-BJGMCSF at different days after infection**

| Virus (MOI 0.25) | oHSV-BJGMCSF | KOS |
|---|---|---|
| the first day | 62±3.5 | 42±2.6 |
| the second day | 41±2.4 | 22±1.3 |
| the third day | 18±1.0 | 7±0.4 |
| the fourth day | 5±0.3 | 0 |

| virus (MOI 0.5) | oHSV-BJGMCSF | KOS |
|---|---|---|
| the first day | 52±3.5 | 33±2.0 |
| the second day | 29±1.6 | 19±1.1 |
| the third day | 11±0.7 | 3±0.2 |
| the fourth day | 0 | 0 |

**Table 7. Survival rate (%) of siHA cells infected with oHSV-BJGMCSF at different days after infection**

| Virus (MOI 0.25) | oHSV-BJGMCSF | KOS |
|---|---|---|
| the first day | 27±1.5 | 18±1.2 |
| the second day | 8±0.5 | 0 |
| the third day | 0 | 0 |

| Virus (MOI 0.5) | oHSV-BJGMCSF | KOS |
|---|---|---|
| the first day | 14±0.8 | 6±0.5 |
| the second day | 1±0.08 | 0 |
| the third day | 0 | 0 |

**Table 8. Survival rate (%) of SK-BR3 cells infected with oHSV-BJGMCSF at different MOIs.**

| Virus (MOI 0.25) | oHSV-BJGMCSF | KOS |
|---|---|---|
| the first day | 24±1.2 | 11±0.8 |
| the second day | 0 | 0 |
| the third day | 0 | 0 |

| Virus (MOI 0.5) | oHSV-BJGMCSF | KOS |
|---|---|---|
| the first day | 8±0.5 | 5±0.3 |
| the second day | 1±0.06 | 0 |
| the third day | 0 | 0 |

**Table 9. Survival rate (%) of ME-180 cells infected with oHSV-BJGMCSF at different days after infection**

| Virus (MOI 0.25) | oHSV-BJGMCSF | KOS |
|---|---|---|
| the first day | 15±0.9 | 9±0.6 |
| the second day | 3±0.2 | 0 |
| the third day | 0 | 0 |

| Virus (MOI 0.5) | oHSV-BJGMCSF | KOS |
|---|---|---|
| the first day | 4±0.3 | 3±0.2 |
| the second day | 1±0.1 | 0 |
| the third day | 0 | 0 |

Tables 6-9 indicates that oHSV-BJGMCSF kills four tumor cells with a similar ability to that seen with KOS. In summary, the results demonstrate that the genetic engineering used to generate the oncolytic viruses mentioned in Example 3 does not significantly affect the ability of the virus and kill tumor cells, and oncolytic viruses oHSV-BJTT and oHSV-BJGMCSF oncolytic viruses provided in the examples of the present disclosure possesses the capacity to kill a variety of tumor cells.

### Experimental example 5

Effect of recombinant oncolytic herpesvirus oHSV-BJTT and oHSV-BJGMCSF on the viability of normal cells

Detection method: Vero cells or primary human corneal epidermal cells were infected with oncolytic viruses oHSV-BJTT, oHSV-BJGMCSF (2 MOI) and wild virus KOS (0.5 MOI). Untreated cells were used as negative control. The viability of the cells infected with oHSV-BJTT and oHSV-BJGMCSF and untreated cells were assayed 3 days after infection, and the viability of the cells infected with wild virus KOS were analyzed 2 days after infection.

Two days after KOS infection, all Vero cells or primary human corneal epithelial cells died, but the viability of the cells infected with oncolytic viruses oHSV-BJTT and oHSV-BJGMCSF was basically the same as that observed for untreated cells (Table 10). This result indicates that the oncolytic viruses oHSV-BJTT and oHSV-BJGMCSF do not affect the viability of normal cells, i.e. the oncolytic viruses are safe to normal cells.

**Table 10. Survival rate (%) of normal cells 3 days after oHSV-BJS or oHSV-BJGMCSF, or 2 days after KOS infection.**

| virus | oHSV-BJTT | oHSV-BJGMCSF | KOS | untreated |
|---|---|---|---|---|
| Vero cells | 96±2 | 95±2 | 0 | 98±2 |
| corneal epithelial cells | 97±12 | 96±12 | 0 | 97±1 |

### Experimental example 6

In order to evaluate the effectiveness and the broad spectrum of oncolytic viruses oHSV-BJTT and oHSV-BJGMCSF in tumor treatment, mouse tumor models for human lung, gastric, liver and colon tumors were established. *In vitro* cultured human non-small cell tumor A549, gastric tumor NCI-N87, and liver cancer SK-HEP-1 cells were subcutaneously injected into BALB/c (lung and gastric tumors) or NPG (liver tumor) mice. When the tumors grew to 800-1000 mm³, they were dissected, cut into 30 mm³ pieces, and then implanted into mice. When the tumors grew to 40-120 mm³, intratumoral injection of oncolytic virus oHSV-BJR was initiated.

The rectal tumor model was established by direct subcutaneous injection of cultured rectal adenocarcinoma HCT-8 cells into a BALB/c mouse. When the tumor grew to 40-120 mm³, intratumoral injection of oncolytic virus oHSV-BJR started.

Intratumoral injection of oncolytic virus oHSV-BJTT or oHSV-BJGMCSF was performed by multiple-point injection once every three days for a total of three times with 2×10⁷ infectious units suspended in 40 µl PBS injected each time. Each group in each model included 8 animals and injection of 40 µl PBS served as a negative control. Tumor volume was measured twice a week after the first virus injection, and the study lasted for 25 to 32 days after the first virus injection depending on when animals in the control group needed to be euthanized. A tumor growth curve of tumor volume over days after the first virus injection was made and the relative inhibition rate calculated by comparing the tumor volume in the test group to the tumor volume in the control group.

The tumor volume of animals in the test group was smaller than that observed in the control group starting from the 8^{th} day or so after the first virus injection (Figure 6. A: lung tumor; B: gastric tumor C: liver tumor; and D: rectal tumor). The difference in tumor volumes between the virus-injected and control groups increased with the days after the first virus injection. Increased with the days after the first virus injection increasing, the inhibition rates of oncolytic virus oHSV-BJTT on the growth of lung, liver, gastric and rectal tumors at the end of study were 44.5, 44.6, 29.7, 57.5%, respectively. And the inhibition rates of oncolytic virus oHSV-BJGMCSF on the growth of lung, liver, gastric and rectal tumors at the end of study were 36.3, 42.2, 24.5, 43.1%, respectively.

The results demonstrate that in various tumor models, the tumor volume in animals injected with oHSV-BJTT or oHSV-BJGMCSF is smaller than that observed for negative control group, indicating that oHSV-BJTT and oHSV-BJGMCSF significantly inhibit the proliferation of various tumors (Figure 6E). The foregoing descriptions are only preferred examples of the present disclosure, and are not intended to limit the present disclosure. Any modification, equivalent replacement, and improvement, etc. made within the concept and principle of the present disclosure shall be included in the protection scope of the present disclosure.

### Industrial applicability

The present disclosure provides a virus and a tumor therapeutic drug for specifically killing tumor cells which can kill a variety of tumor cells effectively while they are safe to non-tumor cells, and have been demonstrated to inhibit tumor growth in animals. Most importantly, the oncolytic viruses retain an intact genome, a basic feature that is strikingly different for currently available oncolytic viruses.

## Claims

1. A virus for specifically killing tumor cells, **characterized in that** the virus is a recombinant oncolytic virus, with an exogenous promoter inserted in the genome, and the exogenous promoter is located upstream of the regulated essential gene of the virus to drive expression of the essential gene in tumor cells but not in normal cells.

2. The virus for specifically killing tumor cells of claim 1, wherein the exogenous promoter is a tumor cells-specific promoter.
Preferably, the tumor -specific promoter is selected from the group consisting of *TERT* promoter, *HER-2* promoter, E2F1 promoter, osteocalcin promoter, *carcinoembryonic antigen* promoter, *survivin* promoter and *ceruloplasmin* promoter.

3. The virus for specifically killing tumor cells of claim 1 or 2, wherein the virus further has an enhancer inserted into the genome, and the enhancer is located between the exogenous promoter and the regulated essential gene to enhance the expression of the essential gene.
Preferably, the enhancer is either a CMV or SV40 enhancer.

4. The virus for specifically killing tumor cells of claim 3, wherein the regulated essential gene(s) in the viral genome can be one or more than one, and the exogenous promoter and the enhancer are located upstream of each essential gene.
Additionally, one or more than one additional copy of each regulated essential gene can be inserted into the viral genome, and the exogenous promoter and the enhancer are located upstream of each copy of the essential gene.

5. The virus for specifically killing tumor cells of claim 3 or 4, wherein the genome further contains an immunostimulatory factor expression sequence and a viral late gene promoter that drives the expression of the immunostimulatory factor expression from the expression sequence, wherein the viral late gene promoter is activated by the gene product of the regulated essential gene.

6. The virus for specifically killing of claim 5, wherein an immunostimulatory factor expressed from the immunostimulatory factor expression sequence is either interleukin 12 or granulocyte-macrophage colony stimulating factor.

7. The virus for specifically killing tumor cells of claim 5 or 6, wherein the virus late gene promoter is the gD promoter when the oncolytic virus is derived from HSV-1 or adenovirus late gene E3 promoter when the oncolytic virus is derived from adenovirus.

8. The virus for specifically killing tumor cells of any one of claims 1-7, wherein the recombinant oncolytic virus is selected from the group consisting of HSV, coxsackie virus, influenza virus, vaccinia virus, measles virus, poliovirus, mumps virus, vesicular stomatitis virus, Newcastle disease virus and adenovirus,
preferably, when the recombinant oncolytic virus is HSV, the essential gene is selected from the group consisting of envelope glycoprotein L, uracil DNA glycosylase, capsid protein, helicase proenzyme subunit, DNA replication initiation binding unwindase, derived protein of myristic acid, deoxyribonuclease, coat serine/threonine protein kinase, DNA packaging terminase subunit 1, coat protein UL16, DNA packaging protein UL17, capsid triplex subunit 2, major capsid protein, envelope protein UL20, nucleoprotein UL24, DNA packaging protein UL25, capsid mature protease, capsid protein, envelope glycoprotein B, single-stranded DNA-binding protein, DNA polymerase catalytic subunit, nuclear egress layer protein, DNA packaging protein UL32, DNA packaging protein UL33, nuclear egress membrane protein, large capsid protein, capsid triplex subunit 1, ribonucleotide reductase subunit 1, ribonucleotide reductase subunit 2, capsule host shutoff protein, DNA polymerase processing subunit, membrane protein UL45, coat protein VP13/14, trans-activating protein VP16, coat protein VP22, envelope glycoprotein N, coat protein UL51, unwindase-primase primase subunit, envelope glycoprotein K, ICP27, nucleoprotein UL55, nucleoprotein UL56, transcription regulation factor ICP4, regulatory protein ICP22, envelope glycoprotein D, and membrane protein US8A;
preferably, when the recombinant oncolytic virus is adenovirus, the essential gene is selected from the group consisting of early protein 1A, early protein 1B 19K, early protein 1B 55K, encapsidation protein Iva2, DNA polymerase, terminal protein precursor pTP, encapsidation protein 52K, capsid protein precursor pIIIa, pentomer matrix, core protein pVII, core protein precursor pX, core protein precursor pVI, hexonmer, proteinase, single-stranded DNA-binding protein, hexamer assembly protein 100K, protein 33K, encapsidation protein 22K, capsid protein precursor, protein U, fibrin, open reading frame 6/7 of regulatory protein E4, regulatory protein E4 34K, open reading frame 4 of regulatory protein E4, open reading frame 3 of regulatory protein E4, open reading frame 2 of regulatory protein E4, and open reading frame 1 of regulatory protein E4;
preferably, when the recombinant oncolytic virus is vaccinia virus, the essential gene is selected from the group consisting of nucleotide reductase small-subunit, serine/threonine kinase, DNA-binding viral core protein, polymerase large-subunit, RNA polymerase subunit, DNA polymerase, sulfhydryl oxidase, hypothetical DNA-binding viral nucleoprotein, DNA-binding phosphoprotein, nucleoid cysteine proteinase, RNA helicase NPH-II, hypothetical metalloproteinase, transcription elongation factor, glutathione-type protein, RNA polymerase, hypothetical viral nucleoprotein, late transcription factor VLTF-1, DNA-binding viral nucleoprotein, viral capsid protein, polymerase small-subunit, RNA polymerase subunit rpo22 depending on DNA, RNA polymerase subunit rpo147 depending on DNA, serine/threonine protein phosphatase, IMV heparin-binding surface protein, DNA-dependent RNA polymerase, late transcription factor VLTF-4, DNA topoisomerase type I, mRNA capping enzyme large-subunit, viral core protein 107, viral core protein 108, uracil-DNA glycosylase, triphosphatase, 70kDa small subunit of early gene transcription factor VETF, RNA polymerase subunit rpo18 depending on DNA, nucleoside triphosphate hydrolase-I, mRNA capping enzyme small-subunit, rifampicin target site, late transcription factor VLTF-2, late transcription factor VLTF-3, disulfide bond forming pathway, precursor p4b of core protein 4b, core protein 39kDa, RNA polymerase subunit rpo19 depending on DNA, 82kDa large subunit of early gene transcription factor VETF, 32kDa small subunit of transcription factor VITF-3, IMV membrane protein 128, precursor P4a of core protein 4a, IMV membrane protein 131, phosphorylated IMV membrane protein, IMV membrane protein A17L, DNA unwindase, viral DNA polymerase processing factor, IMV membrane protein A21L, palmitoyl protein, 45kDa large subunit of intermediate gene transcription factor VITF-3, RNA polymerase subunit rpo132 depending on DNA, RNA polymerase rpo35 depending on DNA, IMV protein A30L, hypothetical ATP enzyme, serine/threonine kinase, EEV mature protein, palmitoylated EEV membrane glycoprotein, IMV surface protein A27L, EEV membrane phosphate glycoprotein, IEV and EEV membrane glycoproteins, EEV membrane glycoprotein, disulfide bond forming pathway protein, hypothetical viral nucleoprotein, IMV membrane protein I2L, poxvirus myristoyl protein, IMV membrane protein L1R, late 16kDa hypothetical membrane protein, hypothetical virus membrane protein H2R, IMV membrane protein A21L, chemokine-binding protein, epidermal growth factor-like protein, and IL-18 binding protein;
preferably, when the recombinant oncolytic virus is coxsackie virus, the essential gene is selected from the group consisting of protein Vpg, core protein 2A, protein 2B, RNA unwindase 2C, protein 3A, proteinase 3C, reverse transcriptase 3D, coat protein Vp4, and protein Vp1;
preferably, when the recombinant oncolytic virus is measles virus, the essential gene is one or more selected from the group consisting of nucleoprotein N, phosphoprotein P, matrix protein M, transmembrane glycoprotein F, transmembrane glycoprotein H, and RNA-dependent RNA polymerase L;
preferably, when the recombinant oncolytic virus is mumps virus, the essential gene is one or more selected from the group consisting of nucleoprotein N, phosphoprotein P, fusion protein F, and RNA polymerase L;
preferably, when the recombinant oncolytic virus is vesicular stomatitis virus, the essential gene is one or more selected from the group consisting of glycoprotein G, nucleoprotein N, phosphoprotein P and RNA polymerase L;
preferably, when the recombinant oncolytic virus is a poliovirus, the essential gene is one or more selected from the group consisting of capsid protein VP1, capsid protein VP2, capsid protein VP3, cysteine protease 2A, protein 2B, protein 2C, protein 3A, protein 3B, proteinase 3C, protein 3D, and RNA-directed RNA polymerase; and
preferably, when the recombinant oncolytic virus is influenza virus, the essential gene is selected from the group consisting of hemagglutinin, neuraminidase, nucleoprotein, membrane protein M1, membrane protein M2, polymerase PA, polymerase PB1-F2, and polymerase PB2.

9. The virus for specifically killing tumor cells of any one of claims 6-8, wherein the recombinant oncolytic virus is HSV type 1, the essential gene is ICP27, and the viral late gene promoter is the gD promoter or the recombinant oncolytic virus is an adenovirus, the essential gene is E1A, and the viral late gene promoter is adenovirus late gene E3 promoter.

10. A tumor therapeutic drug, **characterized by** containing the virus for specifically killing tumor cells of any one of claims 1-9;
preferably, the drug further contains a pharmaceutically acceptable carrier.

11. A nucleic acid sequence, used for preparing the virus of any one of claims 1-9, **characterized in that** the nucleic acid sequence contains a core sequence inserting into the target site of the target virus, and the core sequence comprises a promoter, the target site is located upstream of an essential gene of the target virus, and the promoter is used to drive expression of the essential gene in tumor cells but not in normal cells;
preferably, the promoter is a tumor-specific promoter;
preferably, the tumor-specific promoter is selected from the group consisting of *TERT* promoter, *HER-2* promoter, *E2F1* promoter, *osteocalcin* promoter, *carcinoembryonic antigen* promoter, *survivin* promoter and *ceruloplasmin* promoter.

12. The nucleic acid sequence of claim 11, wherein the target virus is a wild-type lytic virus.

13. The nucleic acid of claim 12, wherein the wild-type lytic virus is selected from the group consisting of wild-type HSV, coxsackie virus, influenza virus, vaccinia virus, measles virus, poliovirus, mumps virus, vesicular stomatitis virus, Newcastle disease virus and adenovirus.

14. The nucleic acid sequence of any one of claims 11-13, wherein the core sequence further comprises an enhancer, and the enhancer is located downstream of the promoter;
preferably, the enhancer is either a CMV or SV40 enhancer;
preferably, the core sequence further comprises an immunostimulatory factor expression sequence and a viral late gene promoter that drives expression of the immunostimulatory factor expression sequence;
preferably, an immunostimulatory factor expressed from the immunostimulatory factor expression sequence is either interleukin 12 or granulocyte-macrophage colony stimulating factor; and
preferably, the viral late gene promoter is the gD promoter when the oncolytic virus is derived from HSV or adenovirus late gene E3 promoter when the oncolytic virus is derived from adenovirus.

15. The nucleic acid sequence of any one of claims 11-14 wherein downstream the tumor specific promoter and enhancer is the open reading frame of an essential gene, followed by a poly(A) sequence. Upstream the tumor specific promoter and downstream the poly(A) sequence are upstream and downstream sequences of the ORF of the regulated essential gene, respectively, which serve as the basis for homologous recombination between the exogenous sequence and viral genome.

16. A vector, **characterized by** containing the nucleic acid sequence of any one of claims 11-15.
